Europäisches Patentamt

European Patent Office

Office européen des brevets

(11) Veröffentlichungsnummer: **0 419 933 A1**

# (12) EUROPÄISCHE PATENTANMELDUNG

(21) Anmeldenummer: 90117471.4

(22) Anmeldetag: 11.09.90

(51) Int. Cl.⁵: **A61B 17/22**

(30) Priorität: 29.09.89 DE 3932516

(43) Veröffentlichungstag der Anmeldung:
03.04.91 Patentblatt 91/14

(84) Benannte Vertragsstaaten:
**DE FR GB IT**

(71) Anmelder: **Richard Wolf GmbH**
**Pforzheimer Strasse 32**
**W-7134 Knittlingen(DE)**

(72) Erfinder: **Belikan, Thomas, Dipl.-Ing.**

Brahmsweg 4
W-7134 Knittlingen(DE)
Erfinder: **Krauss, Werner, Dipl.-Ing.**
Beethovenstrasse 84
W-7134 Knittlingen(DE)
Erfinder: **Wurster, Helmut, Dipl.-Ing.**
Mozartstrasse 20
W-7519 Oberderdingen(DE)

(74) Vertreter: **Wilcken, Thomas, Dipl.-Ing. et al**
Musterbahn 1
W-2400 Lübeck(DE)

(54) **Lithotripsie-Ultraschall-Ortungseinrichtung.**

(57) Es ist eine Lithotripsie-Ultraschall-Ortungsein-richtung beschrieben, die aus mindestens einem, einem Therapiewandler (1) zugeordneten und axial in bezug auf dessen Fokus verstellbaren Ortungs-wandler (2) mit mehreren Fokalbereichen, aus einem Positionsgeber (4), welcher ein den Abstand des Schallkopfes des Ortungswandlers (2) vom Therapie-wandlerfokus (9) repräsentierendes Signal erzeugt, aus einem Rechner (5), aus einem Monitor (6), auf dem ein den Therapiewandlerfokus (9) repräsentie-rendes Zielkreuz darstellbar ist, und aus einer vom Rechner (5) ansteuerbaren Sende-/Empfangselektronik (8) besteht.

Zur Erzeugung eines stets optimalen Bildes des zu zerstörenden Konkrementes steuert der Rechner (5) in Abhängigkeit von dem Signal des Positionsgebers (4) über die Sende-/Empfangselektronik (8) denjenigen Fokalbereich des Ortungswandlers (2) an, welcher bei dem gegebenen momentanen Abstand des Schallkopfes vom Therapiewandlerfokus (9) stets in dessen Bereich liegt. Der Rechner (5) erzeugt darüber hinaus ein das Zielkreuz auf dem Monitor (6) entsprechend dem Signal des Positions-gebers (4) nachführendes Signal, welches dem Mo-nitor (6) zugeführt wird, damit zutreffendenfalls bei jeder Axialstellung des Ortungswandlers (2) das Ab-bild des Konkrements auf dem Monitor (6) in dem Zielkreuz liegt.

FIG. 1

## LITHOTRIPSIE-ULTRASCHALL-ORTUNGSEINRICHTUNG

Die Erfindung betrifft eine Lithotripsie-Ultraschall-Ortungseinrichtung, welche aus mindestens einem, einem Ultraschallstoßwellen aussendenden Therapiewandler zugeordneten und axial in bezug auf dessen Fokus verstellbaren Ortungswandler mit mehreren Fokalbereichen, aus einem Positionsgeber, welcher ein den Abstand des Schallkopfes des Ortungswandlers vom Therapiewandlerfokus repräsentierendes Signal erzeugt, aus einem Rechner, aus einem Monitor, auf dem ein den Therapiewandlerfokus repräsentierendes Zielkreuz darstellbar ist, und aus einer vom Rechner ansteuerbaren Sende-/Empfangselektronik besteht.

Ziel der Anwendung einer gattungsgemäßen Einrichtung ist es, das zu zerstörende Konkrement auf dem Monitor möglichst so abzubilden, daß der behandelnde Arzt hinreichende Informationen über die Lage, Größe und die Beschaffenheit des Steines erhält, um über Art und Dauer der zu applizierenden Ultraschallstoßwellen entscheiden zu können.

Befindet sich die Zielmarke auf dem Monitor dabei auf dem Abbild des Konkrements, so sind der Fokus des Therapiewandlers und das Konkrement koinzident, so daß erst dann die Applikation der Ultraschallstoßwellen sinnvoll ist.

Grundsätzlich ist es vorteilhaft, den Schallkopf des Ortungswandlers, beispielsweise eines B-Scanners, möglichst an die Haut des Patienten heranzuführen, um Reflektionen und Mehrfachechos der von dem Schallkopf ausgesendeten Ultraschallwellen zu vermeiden und um auf diese Weise ein möglichst klares Bild von dem Konkrement zu erhalten. Ausgehend von einer beliebigen Stellung des Ortungswandlers, bei der das Konkrement aber gegebenenfalls aufgrund der erwähnten Mehrfachechos oder Reflektionen nur unklar auf dem Monitor abgebildet ist, kann ein Ortungswandler in Richtung des Fokus des Therapiewandlers verschoben werden, um ein klareres Bild vom Konkrement zu erhalten. Hierbei wandert bei Anwendung eines einfachen B-Scanners das Konkrement aus dem Fokalbereich des Ortungswandlers aus, das heißt die Bildauflösung nimmt ab. Abhilfe schafft hier in der Praxis ein Ortungswandler mit mehreren Fokalbereichen. In Abhängigkeit vom Abstand des Schallkopfes des Ortungswandlers zum Fokus des Therapiewandlers kann per Bedienung eines Betätigungselementes derjenige Fokalbereich des Ortungswandlers ausgewählt werden, in welchem sich momentan das Konkrement befindet, um zu einer bestmöglichen Bildauflösung zu kommen.

Wenn also ein Patient auf einen Behandlungstisch gebettet ist, kann der Ortungswandler an die Haut des Patienten (und damit in Richtung auf den Fokus des Therapiewandlers) verfahren werden. Per manueller Bedienung von Betätigungselementen kann der Arzt dann empirisch den am bestgeeignetsten Fokalbereich auswählen, um eine möglichst gute Bildauflösung zu erzielen. Dies ist auch im Hinblick darauf von Interesse, daß die Konkremente bei unterschiedlichen Indikationen sich in unterschiedlichen Abständen zur Hautoberfläche des Patienten befinden. So liegt beispielsweise ein Harnleiterstein relativ tief im Körper, wohingegen andere Steine dichter an der Hautoberfläche liegen.

Aufgabe der vorliegenden Erfindung ist es daher, die gattungsgemäße Einrichtung so weiterzubilden, daß dem behandelnden Arzt stets ohne Eingriff von außen ein bestmögliches Bild des zu zerstörenden Konkrements auf dem Monitor vermittelt wird, wobei die Zielmarke auf dem Monitor unabhängig von der Axialverstellung des Ortungswandlers stets koinzident mit dem Abbild des Konkrements sein soll, wenn der Therapiewandlerfokus auf das Konkrement ausgerichtet ist.

Gelöst wird diese Aufgabe ausgehend von der Ortungseinrichtung mit den Merkmalen des Oberbegriffs des Anspruches 1 dadurch, daß der Rechner in Abhängigkeit von dem Signal des Positionsgebers über die Sende-/Empfangselektronik denjenigen Fokalbereich des Ortungswandlers ansteuert, welcher bei dem gegebenen momentanen Abstand des Schallkopfes vom Therapiewandlerfokus stets in dessen Bereich liegt, und daß der Rechner ein das Zielkreuz auf dem Monitor entsprechend dem Signal des Positionsgebers nachführendes Signal an den Monitor leitet.

Der Positionsgeber kann beispielsweise fest an dem Getriebe angeordnet sein, mit dessen Hilfe der Ultraschall-Ortungswandler axial verstellbar ist. Der Positionsgeber erzeugt ein Signal, welches den jeweiligen Abstand des Schallkopfes des Ultraschall-Ortungswandlers zum Fokus des Therapiewandlers repräsentiert. Dieses Signal wird dem Rechner zugeführt. Aufgrund der festen Zuordnung des Ultraschall-Ortungswandlers zu dem Therapiewandler und damit zu dessen Fokus ist der Rechner in der Lage, zu ermitteln, welcher Fokalbereich des Ultraschall-Ortungswandlers im Bereich des abzubildenden Konkrements liegt. Der Rechner erzeugt ein dementsprechendes Ausgangssignal, welches einer Sende-/Empfangselektronik zugeführt wird, welche ihrerseits diejenigen aktiven und passiven Bereiche des Ortungswandlers ansteuert, welche dem ausgewählten Fokalbereich entsprechen. Der Rechner erzeugt im übrigen ein weiteres Signal, welches die Zielmarke auf dem Monitor entsprechend dem Signal des Positionsgebers und damit entsprechend dem momentanen Abstand des Schallkopfes des Ultraschall-Ortungswandlers vom Therapiewandlerfokus nachführt.

Der Ortungswandler kann vorteilhafterweise einen Schallkopf mit mindestens zwei Kristallringen aufweisen. Dementsprechend weist dieser Schallkopf mindestens zwei Fokalbereiche auf. Ein derartiger Ortungswandler arbeitet nach dem sogenannten "Annular-Phased-Array"-Prinzip.Derartige Schallköpfe haben gegenüber beispielsweise den "Linear-Phased-Array" -Schallköpfen den Vorteil, daß sie eine symmetrische Schallkeule abstrahlen. Bei der Verwendung von "Linear-Phased-Array" -Schallköpfen kann das Schallfeld quer zur Schnittebene elektronisch nicht kontrolliert werden. "Annular-Phased-Array"-Schallköpfe können hingegen auch dünne Schichten senkrecht zur Schnittebene erfassen, da der Schallstrahl kreissymmetrisch formiert ist. Vorteilhaft ist es weiterhin, wenn der Ortungswandler zusätzlich drehbar um seine Hauptachse gelagert ist. Hierdurch ergibt sich die Möglichkeit, das Konkrement aus verschiedenen Bildebenen auf dem Monitor darzustellen, so daß sich der Informationsgehalt des Bildes erhöht.

Besonders einfach und vorteilhaft ist eine Ausführungsform der erfindungsgemäßen Einrichtung, wenn der Therapiewandler ein kalottenförmiger Wandler ist und wenn der bzw. die Ortungswandler im Inneren der Kalotte angeordnet sind. Hierdurch ergibt sich eine einfache feste Zuordnung der Ortungswandler zum Fokus des Therapiewandlers.

Mit der vorbeschriebenen Einrichtung wird dem behandelnden Arzt eine Einrichtung an die Hand gegeben, die automatisch stets für die bestmögliche Abbildung des Konkrements auf dem Monitor sorgt. Wird der Ortungswandler näher an den Therapiewandlerfokus herangeführt, das heißt also an die Haut des Patienten herangeführt, so wird automatisch auf den dann optimalen Fokalbereich des Schallkopfes umgeschaltet und die Zielmarke auf dem Monitor so nachgeführt, daß das Konkrement zutreffendenfalls als im Fokus des Therapiewandlers befindlich dargestellt wird.

Die Erfindung wird anhand eines Ausführungsbeispieles gemäß der Figuren näher erläutert. Hierbei zeigt:

Figur 1 die Ortungseinrichtung, wobei zwei Ortungswandler in einem kalottenförmigen Therapiewandler angeordnet sind, und

Figur 2 eine vergrößerte Ansicht eines Schallkopfes der Ultraschall-Ortungswandler.

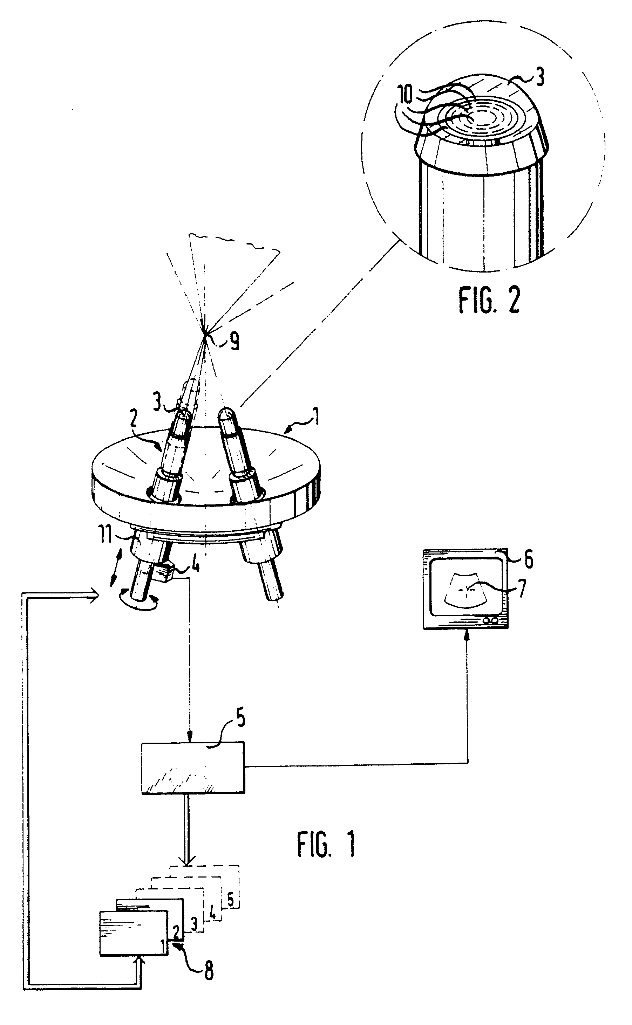

Gemäß Figur 1 sind zwei Ortungswandler 2 fest in der Kalotte des Therapiewandlers 1 angeordnet, dergestalt, daß sie mittels eines Antriebes 11 axial verstellbar in bezug auf den Fokus 9 des Therapiewandlers 1 verstellbar sind, wie dies durch den Doppelpfeil angedeutet ist. Zusätzlich hierzu sind die Ortungswandler 2 um ihre Hauptachse drehbar gelagert.

Mit dem Antrieb 11 ist für den links dargestellten Ortungswandler 2 ein Positionsgeber 4 verbunden. Der Positionsgeber 4 kann beispielsweise aus einem Präzisionspotentiometer oder aus einem digitalen Absolutwertgeber bestehen. In jedem Falle erzeugt der Positionsgeber 4 ein Signal, welches den Abstand des Schallkopfes 3 vom Fokus 9 des Therapiewandlers 1 repräsentiert. Die Eindeutigkeit des erzeugten Signals resultiert aus der festen Zuordnung des Ortungswandlers 2 zum Therapiewandler 1.

Das Signal des Positionsgebers 4 wird dem Rechner 5 zugeführt, der es weiterverarbeitet.

Einerseits erzeugt der Rechner 5 ein Steuersignal, welches der Sende-/Empfangselektronik 8 zugeleitet wird. Aufgrund des Signals vom Positionsgeber 4 "erkennt" der Rechner 5, in welchem Abstand sich der Schallkopf 3 vom Fokus 9 des Therapiewandlers 1 befindet. Dementsprechend steuert er die entsprechende Ebene der Sende-/Empfangselektronik 8 an, welche vorliegend schematisch als aus fünf Ebenen bestehend dargestellt ist.

Die Sende-/Empfangselektronik 8 weist so viele Ebenen auf, wie der Schallkopf 3 Kristallringe 10 aufweist. Die Kristallringe 10 im Schallkopf 3 arbeiten hier nach dem Annular-Phased-Array-Prinzip.

Andererseits erzeugt der Rechner 5 ein Signal, welches die Zielmarke 7 auf dem Monitor 6 entsprechend dem Signal des Positionsgebers 4 und damit entsprechend dem momentanen Abstand des Schallkopfes 3 vom Fokus 9 des Therapiewandlers 1 nachführt.

Ausgehend von der Situation, daß das zu zerstörende Konkrement im Patientenkörper grob geortet worden ist und der Fokus 9 auf das Konkrement ausgerichtet ist, wird der Ortungswandler 2 aus den weiter oben dargelegten Gründen an die Haut des Patienten herangeführt, das heißt also in Richtung des Fokus 9 bewegt. Die erläuterte Kompensation durch das vom Rechner 5 erzeugte Nachführsignal für die Zielmarke 7 sorgt dafür, daß diese sich stets auf dem Abbild des Konkrements befindet, sofern sich dieses tatsächlich noch im Fokus 9 des Therapiewandlers 1 befindet. Das Nachführsignal vom Rechner 5 wird in bekannter Weise den Ablenkeinrichtungen des Monitors 7 zugeführt.

## Ansprüche

1. Lithotripsie-Ultraschall-Ortungseinrichtung, bestehend aus mindestens einem, einem Therapiewandler zugeordneten und axial in bezug auf dessen Fokus verstellbaren Ortungswandler mit mehreren Fokalbereichen, einem Positionsgeber, welcher ein den Abstand des Schallkopfes des Ortungswandlers vom Therapiewandlerfokus repräsentierendes Signal erzeugt, einem Rechner, einem Monitor, auf dem ein den Therapiewandlerfokus repräsentierendes Zielkreuz darstellbar ist, und einer vom Rechner ansteuerbaren Sende-/Empfangselektronik, dadurch gekennzeichnet, daß der Rechner (5) in Abhängigkeit von dem Signal des Positionsgebers (4) über die Sende-/Empfangselektronik(8) denjenigen Fokalbereich des Ortungswandlers (2) ansteuert, welcher bei dem gegebenen momentanen Abstand des Schallkopfes(3) vom Therapiewandlerfokus (9) stets in dessen Bereich liegt, und daß der Rechner (5) ein das Zielkreuz(7) auf dem Monitor (6) entsprechend dem Signal des Positionsgebers (4) nachführendes Signal an den Monitor (6) leitet.

2. Lithotripsie-Ultraschall-Ortungseinrichtung nach Anspruch 1, dadurch gekennzeichnet, daß der Ortungswandler (2) einen Schallkopf (3) mit mindestens zwei Kristallringen (10) aufweist.

3. Lithotripsie-Ultraschall-Ortungseinrichtung nach Anspruch 1 oder 2, dadurch gekennzeichnet, daß der Ortungswandler(2) zusätzlich drehbar um seine Hauptachse gelagert ist.

4. Lithotripsie-Ultraschall-Ortungseinrichtung nach einem der Ansprüche 1 bis 3, dadurch gekennzeichnet, daß der Therapiewandler (1) ein kalottenförmiger Wandler ist und daß der bzw. die Ortungswandler (2) im Inneren der Kalotte angeordnet sind.

FIG. 2

FIG. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-2 619 003 (TOSHIBA)<br>* Seite 8, Zeile 10 - Seite 9, Zeile 19; Figur 2 *<br>– – – | 1-4 | A 61 B 17/22 |
| Y | US-A-4 537 074 (DIETZ)<br>* Spalte 2, Zeilen 23-43; Figur 3 *<br>– – – | 1-4 | |
| A | DE-A-3 119 295 (SIEMENS)<br>* Seite 7, Zeilen 14-21; Seite 9, Zeilen 17-36; Figur 1 *<br>– – – | 1 | |
| A | EP-A-0 194 897 (TOSHIBA)<br>* Seite 5, Zeile 31 - Seite 6, Zeile 28; Figuren 2,3 *<br>– – – – – | 1,2 | |
| | | | **RECHERCHIERTE SACHGEBIETE (Int. Cl.5)**<br><br>A 61 B<br>A 61 F<br>G 10 K |

Der vorliegende Recherchenbericht wurde für alle Patentansprüche erstellt

| Recherchenort | Abschlußdatum der Recherche | Prüfer |
|---|---|---|
| Den Haag | 04 Januar 91 | MOERS R.J. |